# EUROPEAN PATENT SPECIFICATION

(11) **EP 3 541 340 B1**
(45) Date of publication and mention of the grant of the patent: **05.03.2025**
(21) Application number: 17818316.6
(22) Date of filing: 13.11.2017
(51) Int. Cl.: A61G 10/02, A61F 7/00, F25D 3/10

(54) **CONTAINER**
GROSSRAUMBEHÄLTER
CONTENEUR

(30) Priority: 18.11.2016 PL 41952216
(43) Date of publication of application: 25.09.2019
(73) Proprietor: Creator Spólka Z Ograniczona Odpowiedzialnosia, 54-154 Wroclaw (PL)
(72) Inventor: JÓZEFOWICZ, Adam, 54-130 Wroclaw (PL)
(74) Representative: Rejman, Tadeusz
(86) International application number: PCT/PL2017/000114
(87) International publication number: WO 2018/093280

(56) References cited:
- WO-A1-03/039414
- AT-B- 135 813
- DE-A1- 3 441 091
- PL-B1- 213 499
- PL-Y1- 66 743
- US-A- 1 471 144
- CREATOR SP. Z O.O.: "OFFER FOR THE CONSTRUCTION OF A SYSTEMIC CRYOTHERAPY CHAMBER OFFER FOR THE CONSTRUCTION OF A SYSTEMIC CRYOTHERAPY CHAMBER Offer for the Construction of a Systemic Cryotherapy Chamber 3 CREATOR Sp CONTENTS", 20 December 2015 (2015-12-20), http://kriokomory.pl, XP055447394, Retrieved from the Internet <URL:https://web.archive.org/web/20151220021244if_/http://kriokomory.pl/doc/oferta_en.pdf> [retrieved on 20180202]
- ADRIANA BORUSZEWSKA: "Jedyna taka w Polsce. Mobilna kriokomora zaparkowala na AWF-ie - Gazetawroclawska.pl", 21 October 2016 (2016-10-21), XP055447463, Retrieved from the Internet <URL:http://www.gazetawroclawska.pl/wiadomosci/a/jedyna-taka-w-polsce-mobilna-kriokomora-zaparkowala-na-awfie,10768608/> [retrieved on 20180202]

## Description

The object of the invention is a container intended for moving to different places and in particular to places of the sporting events taking place.

A patent description no. PL/EP 2744731 discloses a reversible folding transport container. This container comprises a roof structure, a floor structure in front of the roof structure, side wall structures between the floor structure and the roof structure. Each of the side wall structures has an outer surface and an inner surface. The container also contains a front wall and a rear wall. The rear wall is equipped with doors.

A patent description no. PL 215175 discloses a refrigerated container with controlled internal atmosphere. This container is intended for long-term storage of various types of fruits and vegetables, especially raspberries, forest berries, American blueberries, plums, apples, cauliflowers, tomatoes, mushrooms and peppers in fresh and raw state. This container has a box with air circulation and control system, temperature sensors and air cooler. The storing part has insulated walls. The floor of the box is equipped with roller guides with locks and its front wall is connected to a chamber housing a set of refrigeration equipment and atmosphere control installation inside the box of this container. The refrigeration equipment set consists of a refrigerating condensing unit equipped with a temperature sensor located in the box. The refrigerating condensing unit is connected by piping and electric cable with suction filter, elastic coupling, shut-off valve, sight glass, drainage filter and high pressure switch. In addition, the container has a control cabinet with the air cooler. A set of atmosphere control devices consists of a nitrogen generator, ethylene scrubbers, CO₂ and oxygen scrubbers connected with piping to the inside of the box.

The publication entitled 'Offer for the construction of a systemic cryotherapy chamber offer for the construction of a systemic cryotherapy chamber' dated 20/12/2015 (see https://web.archive.org/web/20151220021244if_/http://kriokomory.pl/doc/oferta_ en.pdf) discloses the preamble of claim 1, disclosing a cryogenic chamber consisting of a vestibule and an actual chamber, where the walls are filled with thermal insulation. Inside the chamber there are lime-wood panels. A cooling agent used is air at a temperature of - 110°C to -120 °C. The cooling factor is liquid nitrogen. Treatment temperature is programmed in range -80°C to -160°C every 5 °C. A vestibule temperature is - 60 °C ± 5 °C.

The publication in newspaper Gazetawroclawska.pl entitled 'Jedyna taka w Polsce. Mobilna kriokomora zaparkowala na AWF-ie' (see Adriana Boruszewska, http://www.gazetawroclawska.pl/wiadomosci/a/jedyna-taka-w-polsce-mobilna-kriokomora-zaparkowala-na-awfie) discloses information about a mobile cryochamber. Treatment in this cryochamber lasts from 2 to 3 minutes in the operating temperature range from -120°C to -160°C. The mobile chamber is intended primarily for those who practice multi-stage sports, such as cycling or skiing.

The document PL 66 743 Y1 discloses a cryogenic chamber comprising a refrigeration unit and the walls which are formed of segments releasably connected to each other. The individual segments are connected with two pairs of locks. The walls have a hard foam insulation. This cryogenic chamber is designed to accommodate people undergoing therapeutic treatments in low temperatures below -110 °C.

The invention is as defined in claim 1.

The container according to the invention allows the use of therapeutic treatments of cryotherapy in any conditions, and in particular in places of the sporting events taking place. The only conditions are: transport of the container and nitrogen tank and supply of electricity. The container according to the invention allows for a significant expansion of the therapeutic services in the form of cryotherapy and to reduce the cost of these services. The container with a cryochamber can be used in small towns far away from medical centers, which eliminates the need for costly and time-consuming commuting to cryotherapy treatments.

The container according to the invention is further explained in the embodiment and in the drawing in which Fig. 1 shows a general view of the container; Fig. 2 shows a top view of the interior of the container skipping its roof; Fig. 3 is a flow chart of the cryochamber supply, Fig. 4 is a cross section through the cryochamber wall.

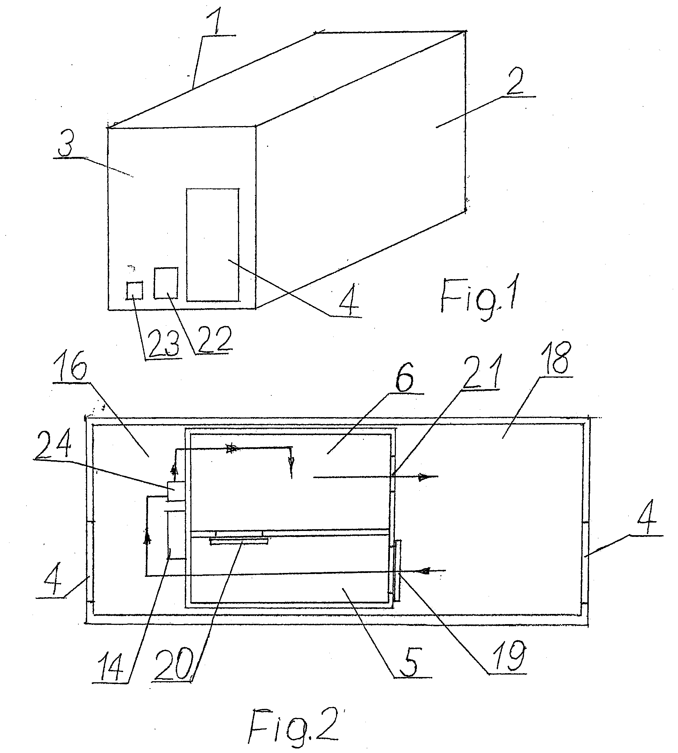

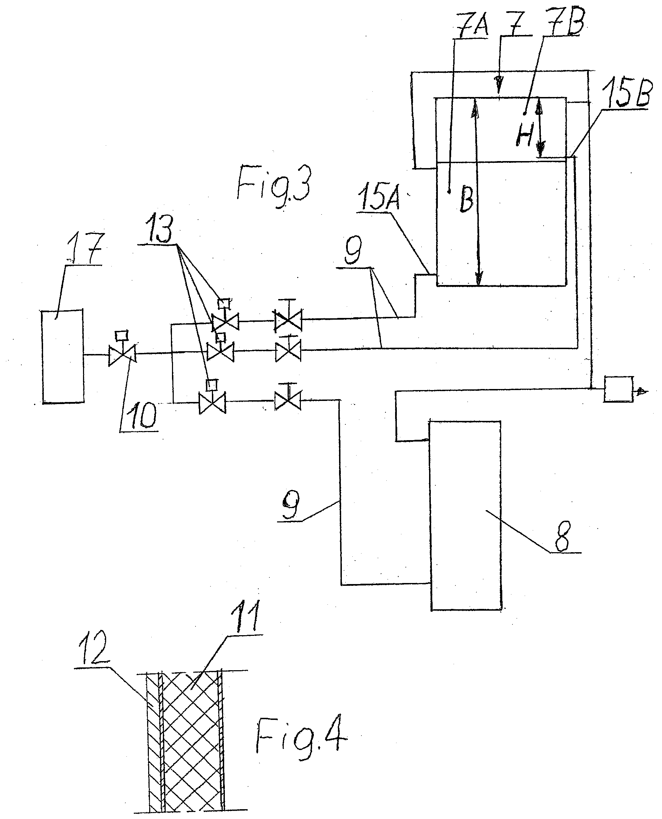

As illustrated in Fig1 - Fig. 4, the container according to the invention is formed of a spatial rectangular metal frame 1 to which the walls 2 and the floor (not shown) are attached. Doors 4 are mounted in both front walls 3. The container has two internally insulated chambers, one of which is an atrium 5 and the other is a cryochamber 6 equipped with a cooling system for temperatures from -120 to -160°C. The cooling system consists of: one bisectional 7A and 7b finned heat exchanger 7 placed in the cryochamber 6A and the atrium is provided with one heat exchanger 8. Each heat exchanger 7 and 8 is connected by ducts 9 to the main valve 10 located at the front wall 3. Insulated chambers 5 and 6 have polyurethane insulated 11 walls and they have a wooden insulation 12 from the inside as shown in Fig.4. Insulated chambers are provided with air supply. Heat exchangers 7 and 8 are connected to the main valve 10 via said ducts 9 and electrovalves 13 connected by electric cables to the control cabinet 14 located on one of the container walls. The cryochamber 6 heat exchanger 7 has nitrogen supply pipes 15 located: one pipe is located at the bottom of the exchanger 7 and the second pipe 15B is located at the top of the exchanger 7. The pipe 15B is located at a distance H from the upper surface of the exchanger 7 of 0.35 of total B heat exchanger 7 height.

The use of the container is as follows. The person operating the container and thus the cryochamber enters the room 16 and switches on the control apparatus located in the control cabinet 14. Both the control cabinet 14 and the entire control apparatus is typical of stationary cryogenic chambers and therefore its description is omitted. The control apparatus activates the valve 10 and the valves 13, then the nitrogen flows from the tank 17 to the heat exchangers 7 and 8. At the same time the control apparatus measures the temperature in the atrium 5 and the cryochamber 6. Once the set temperature has been reached, the patient to be treated enters the dressing room 18, where he has the lockers, the bench, the chairs and hangers, undresses and enters the atrium 5 through door 19. Door 19 is equipped with a window to observe the patient. Then the patient enters the cryochamber 6 through door 20 where is being treated, still being observed through the window 21. The cryochamber 6 is equipped with typical alarm and emergency devices so that the staff would intervene in the case a patient was not feeling good. Tank 17 containing liquefied nitrogen is located outside the container and is connected to an installation placed in the container via a connector 22 attached to the wall 3. Similarly, an electrical connector 23 which supplies the control cabinet 14 and the entire apparatus contained in the container is attached to the wall 3. The cryochamber 6 is provided with a supply of dried air. The air is taken from the dressing room 18 and flows through the ducts to the dryer 24. When dried, the air is fed to the heat exchangers 7A and 7B, from which the dried and cooled air enters the cryochamber 6. The air circulation diagram is illustrated with arrows in Fig. 2.

## Claims

1. A container formed of a spatial, cuboidal metal frame to which the walls and floor are attached and doors are fixed in both front walls,
the container comprising internally insulated chambers (5, 6) having polyurethane insulated (11) walls and having a wooden insulation (12) from the inside,
one of which is an atrium(5) and the other is a cryochamber (6) equipped with a cooling system for temperatures from -120 to -160°C **characterized in that** a cooling system consists of: one bisectional (7A, 7B) heat exchanger (7) placed in the cryochamber (6) and one heat exchanger (8) located in the atrium (5) and each heat exchanger (7, 8) is connected by ducts (9) to a main valve (10) located at the front wall (3) of the container and the insulated cryochamber (6) is provided with a supply of dried air,
wherein heat exchangers (7 and 8) are connected to the main valve (10) via said ducts and electrovalves (13) connected by electric cables to a control cabinet (14) located on one of the container walls and the cryochamber (6) heat exchanger section (7A) has nitrogen supply pipes (15A) located at the bottom of the exchanger (7) and the second section (7B) at the top of the exchanger (7) and the heat exchanger (7) section (7B) has a nitrogen supply pipe (15B) at a distance (H) from the upper surface of the exchanger (7) of 0.3 - 0.4 of total heat exchanger (7) height (B).

## Patentansprüche

1. Behälter, ausgebildet aus einem räumlichen, rechteckigen Metallrahmen, an dem Wände und ein Boden angebracht sind und Türen in den beiden Stirnwänden befestigt sind, wobei der Behälter im Inneren isolierte Kammern (5, 6) enthält, die Wände mit einer Polyurethan-Isolierung (11) aufweisen und von innen eine Holzisolierung (12) aufweisen, von denen eine eine Vorkammer (5) und die andere eine mit einer Kühlanlage für Temperaturen von -120 bis -1600 °C ausgestattete Kryokammer (6) ist, **dadurch gekennzeichnet, dass** die Kühlanlage aus Folgendem besteht: einem Wärmetauscher (7) mit zwei Abschnitten (7A, 7B), der sich in der Kryokammer (6) befindet, und einem Wärmetauscher (8), der sich in der Vorkammer (5) befindet, und jeder der Wärmetauscher (7, 8) ist durch Leitungen (9) mit dem Hauptventil (10) verbunden, das sich an der Vorderwand (3) des Behälters befindet, und die isolierte Kryokammer (6) wird mit trockener Luft versorgt, wobei jeder Wärmetauscher (7, 8) über die Leitungen mit dem Hauptventil (10) und den Magnetventilen (13) über elektrische Kabel mit dem Steuerschrank (14) verbunden ist, der sich an einer der Wände des Behälters befindet, und der Abschnitt (7A) der Kryokammer (6) des Wärmetauschers mit Stickstoff über Rohre (15A) versorgt wird, die sich im unteren Teil des Wärmetauschers (7) befinden, und für den zweiten Abschnitt (7B) im oberen Teil des Wärmetauschers (7), und der Abschnitt (7B) des Wärmetauschers (7) einen Stutzen (15B) hat, der Stickstoff in einem Abstand (H) von der Oberseite des Wärmetauschers (7) von 0,3 - 0,4 der Gesamthöhe (B) des Wärmetauschers (7) zuführt.

## Revendications

1. Un conteneur formé d'un cadre métallique rectangulaire spatial auquel sont fixés des parois et un plancher, ainsi que des portes sur les deux parois frontales, le conteneur contenant des chambres isolées à l'intérieur (5,6) avec une isolation en polyuréthane (11) des parois et une isolation en bois (12) à l'intérieur, dont l'une est une antichambre (5) et l'autre une cryochambre (6) équipée d'une installation de refroidissement pour des températures allant de - 120 à -160°C, **caractérisé en ce que** l'installation de refroidissement est constituée de : un échangeur de chaleur (7) à deux sections (7A, 7B) placé dans la cryochambre (6) et un échangeur de chaleur (8) placé dans l'antichambre (5), et chacun des échangeurs de chaleur (7, 8) est relié par des tuyaux (9) à la vanne principale (10) située au niveau de la paroi avant (3) du conteneur et la cryochambre isolée (6) est alimentée en air sec, où chacun des échangeurs de chaleur (7, 8) est relié par lesdits tuyaux à la vanne principale (10) et aux électrovannes (13) au moyen de câbles électriques à l'armoire de commande (14) située sur l'une des parois du conteneur et la cryochambre (6) section (7A) de l'échangeur de chaleur est alimentée en azote par des tuyaux (15A) situés dans la partie inférieure de l'échangeur de chaleur (7) et la seconde section (7B) dans la partie supérieure de l'échangeur de chaleur (7) et la section (7B) de l'échangeur de chaleur (7) a un embout (15B) fournissant de l'azote à une distance (H) de la surface supérieure de l'échangeur de chaleur (7) de 0,3 - 0,4 de la hauteur totale (B) de l'échangeur de chaleur (7).
